# EUROPÄISCHE PATENTSCHRIFT

(11) **EP 0 638 076 B1**
(45) Veröffentlichungstag und Bekanntmachung des Hinweises auf die Patenterteilung: **16.09.1998**
(21) Anmeldenummer: 93920533.2
(22) Anmeldetag: 16.03.1993
(51) Int. Cl.: C07D 291/06

(54) **VERFAHREN ZUR HERSTELLUNG DER NICHT-TOXISCHEN SALZE DES 6-METHYL-3,4-DIHYDRO-1,2,3-OXATHIAZIN-4-ON-2,2-DIOXIDS UND ANORDNUNG ZUR DURCHFÜHRUNG DIESES VERFAHRENS**
PROCESS AND DEVICE FOR PREPARING THE NON-TOXIC SALTS OF 6-METHYL-3,4-DIHYDRO-1,2,3-OXATHIAZIN-4-ON-2,2-DIOXIDE
PROCEDE ET DISPOSITIF DE PREPARATION DES SELS NON TOXIQUES DU 6-METHYL-3,4-DIHYDRO-1,2,3-OXATHIAZIN-4-ON-2,2-DIOXYDE

(30) Priorität: 17.03.1992 DE 4208513
(43) Veröffentlichungstag der Anmeldung: 15.02.1995
(73) Patentinhaber: HOECHST AKTIENGESELLSCHAFT, 65926 Frankfurt am Main (DE)
(72) Erfinder: ROSCHER, Günter, D-6233 Kelkheim (DE); LITTERER, Heinz, D-6208 Bad Schwalbach (DE); ENGELMANN, Axel, D-6240 Königstein (DE); KAUFMANN, Wolf-Dietmar, D-6242 Kronberg (DE); LAUGWITZ, Bernd, D-6232 Bad Soden (DE); SCHNABEL, Hans-Dietmar, D-6239 Eppstein (DE)
(74) Vertreter: Schweitzer, Klaus, Dr.
(86) Internationale Anmeldenummer: EP9300606
(87) Internationale Veröffentlichungsnummer: WO9319055

(56) Entgegenhaltungen:
- EP-A- 0 155 634
- EP-A- 0 159 516
- EP-A- 0 217 024
- EP-A- 0 218 076
- ANGEWANDTE CHEMIE. INTERNATIONAL EDITION. Bd. 12, Nr. 11, November 1973, WEINHEIM DE Seiten 869 - 942 K. CLAUSS, H. JENSEN 'OXATHIAZINONE DIOXIDES - A NEW GROUP OF SWEETENING AGENTS.'

## Beschreibung

6-Methyl-3,4-dihydro-1,2,3-oxathiazin-4-on-2,2-dioxyd (nachfolgend Acesulfam-H) wird seit einiger Zeit in Form seines Kaliumsalzes (nachfolgend Acesulfam-K) 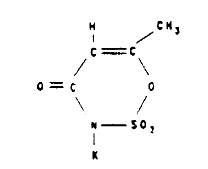 wegen seines intensiven Süßgeschmackes als Süßstoff auf dem Nahrungsmittelsektor verwendet.

Für die Herstellung von Acesulfam-K ist eine Reihe verschiedener Verfahren bekannt (vgl. u.a. Angewandte Chemie, 22(1973), Seiten 965 bis 973). Von besonderem Interesse ist heute ein Verfahren, bei dem in einem Lösungsmittel, vorzugsweise Methylenchlorid, zunächst Amidosulfonsäure, vorzugsweise jedoch ein lösliches Salz der Amidosulfonsäure, mit Diketen zur Acetoacetamidoverbindung umgesetzt wird. Als Salze der Amidosulfonsäure werden dabei im allgemeinen Alkali- oder Ammoniumsalze, vorzugsweise Trialkylammoniumsalze, verwendet. Bei der Umsetzung mit Diketen entsteht dabei das Acetoacetamidosulfonsäuresalz (I) entsprechend der Reaktionsgleichung 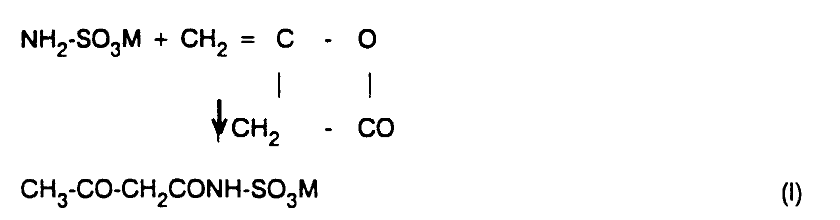 (M = Basenkation, insbesondere HN(Alkyl C₁-C₆)₃)

Die vorzugsweise in Methylenchlorid gelöste Acetoacetamidoverbindung (I) wird dann unter bestimmten Reaktionsbedingungen mit einer Lösung von SO₃, vorzugsweise in Methylenchlorid, zur Reaktion gebracht und dabei cyclisiert; das SO₃ wird dabei vorzugsweise im Überschuß eingesetzt (vgl. hierzu die EP-Offenlegungsschriften 155.634, 159.516, 217.076 und 218.076). Auf diese Weise entsteht ein Cydisierungsprodukt, aus dem durch Umsetzung mit Wasser (Hydrolyse) in einem Hydrolysegefäß Acesulfam-H, die sogenannte "Süßstoffsäure" (II), entsteht: 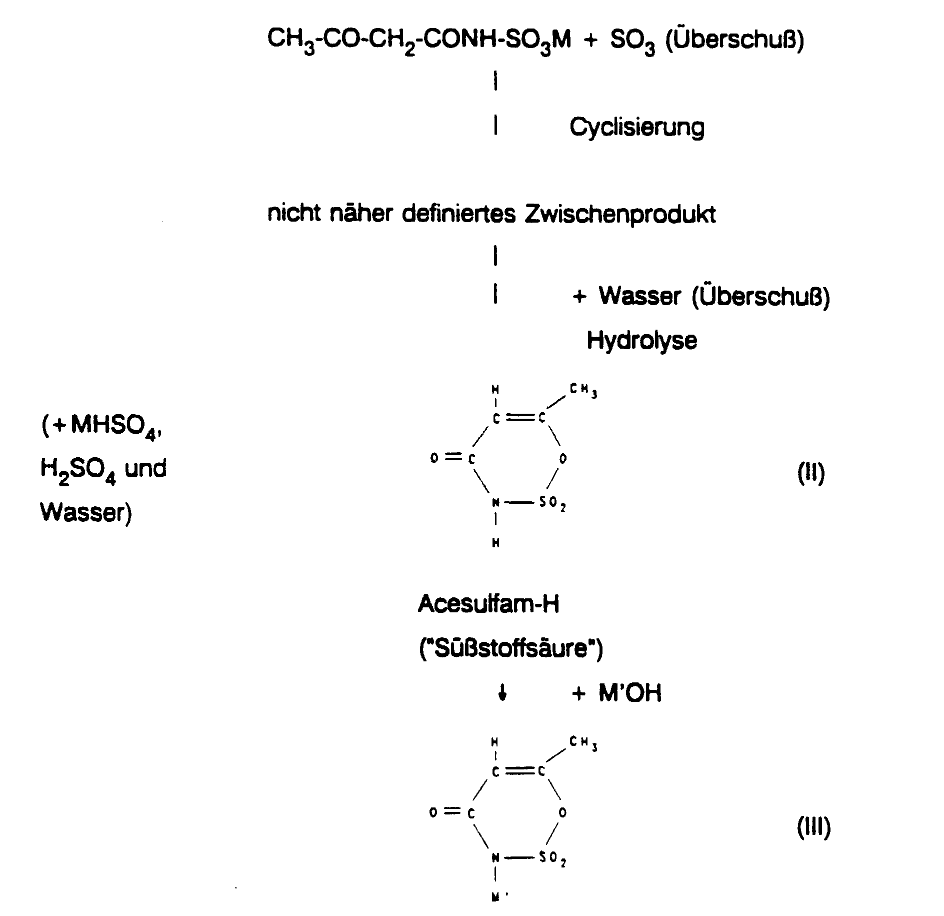 Acesulfam - Salz (Acesulfam-K)
(M' = Basenkation, vorzugsweise K)

Sowohl die Cyclisierung wie auch die Hydrolyse sind dabei schnell verlaufende, stark exotherme Reaktionen.

Das Reaktionsgemisch trennt sich in eine organische Phase, vorzugsweise Methylenchloridphase und eine wäßrige Schwefelsäurephase. Die Süßstoffsäure (II) befindet sich zum größten Teil (etwa 4/5) in der organischen Phase, etwa 1/5 ist in der Schwefelsäurephase gelöst. Die Schwefelsäurephase enthält nahezu die Gesamtmenge des Alkalis oder Ammoniums, vorzugsweise Trialkylamins als Sulfat, vorzugsweise Trialkylammoniumhydrogensulfat. Die Schwefelsäurephase kann nach Abstrippen von noch vorhandenem Lösungsmittel, vorzugsweise Methylenchlorid, an anderer Stelle weiter verwertet werden.

Die Abtrennung der Süßstoffsäure in Form des gewünschten Salzes (III) erfolgt zweckmäßig nach der in genannten EP-Offenlegungsschrift 218.076 beschriebenen Methode. Dabei wird zunächst mit einem Lösungsmittel, vorzugsweise Methylenchlorid, aus der Schwefelsäurephase die darin enthaltene Süßstoffsäure in einem Extraktionsgefäß extrahiert. Das hier verwendete Lösungsmittel wird mit der normalen Lösungsmittelphase, die den größten Teil der Süßstoffsäure enthält, vereinigt. Aus den vereinigten Lösungsmittelphasen wird dann mit wenig Wasser das in geringer Menge in der Lösungsmittelphase gelöste Sulfat in einem Extraktionsgefäß extrahiert und mit dem Extraktionswasser zurückgeführt zur Umsetzung des Cyclisierungsprodukts im Hydrolysegefäß mit Wasser zur Süßstoffsäure. Aus der verbleibenden, weitgehend sulfatfreien Lösung der Süßstoffsäure im Lösungsmittel wird die Süßstoffsäure in Form des gewünschten Salzes, vorzugsweise des Kaliumsalzes, mit wässriger Lauge, vorzugsweise Kalilauge, herausgeholt. Die wäßrige Lösung des Süßstoffes (III) kann nach gängigen Methoden, zum Beispiel durch Eindampfen oder Ausfällen mit Fremdlösungsmitteln, zu dem reinem kristallisierten Süßstoff (III) aufgearbeitet werden.

Das verbleibende Lösungsmittel ist wassergesättigt und enthält einen großen Teil der bei der Reaktion gebildeten Nebenprodukte, wie Aceton, Trialkylamin und Trialkylammoniumsalz der Süßstoffsäure im Falle des Einsatzes des Trialkylammoniumsalzes der Amidosulfonsäure, undefinierte Hochsieder und gelöste Harze.

Das so verunreinigte Lösungsmittel kann in dieser Form nicht wieder eingesetzt werden. So bilden sich bei der Mischung mit dem SO₃ in der Cyclisierungsstufe mit den Verunreinigungen dunkel gefärbte, feste, nicht lösliche Verbindungen, die zu Verstopfungen in den Leitungen führen. Mit Aceton treten daneben gefärbte Folgeprodukte auf, die zur Süßstofflösung durchschlagen. Aus solchen Lösungen läßt sich kein farbloser Süßstoff mehr abtrennen. Auch bei der Herstellung der Acetoacetamidoverbindung (I) aus dem Amidosulfonsäuresalz und Diketen kann dieses verunreinigte Lösungsmittel nicht verwendet werden, weil die Ausbeute an Acetoacetamidoverbindung absinkt und stark gefärbte Nebenprodukte gebildet werden. Diese werden bis in die später gewonnene Süßstofflösung hineingeschleppt und verhindern die Abtrennung von farblosem, festen Acesulfam-Salz (III) aus der Lösung.

Das zur Mischung mit SO₃ und zur Herstellung der Acetoacetamidoverbindung verwendete Lösungsmittel darf deshalb nur geringe Mengen an Verunreinigungen enthalten. Die Rückgewinnung von reinem Lösungsmittel aus dem rohen Lösungsmittel, das hinter der Neutralisation der Süßstoffsäure in Form ihres Salzes anfällt, erfordert einen hohen apparativen Aufwand mit beträchtlichem Energieverbrauch, weil bei der Synthese aus Gründen der Ausbeute und der begrenzten Löslichkeit der Süßstoffsäure im Lösungsmittel nur mit sehr verdünnten Lösungen gearbeitet werden kann. Die Konzentration der Süßstoffsäure beispielsweise in der Methylenchloridlösung, die dann zur Neutralisation mit der Lauge geht, liegt in der Regel nur bei etwa 2 bis 3 Gew.-%. Demgemäß müssen bei der destillativen Reinigung des rohen Lösungsmittels zur Gewinnung von reinem wieder einsetzbarem Lösungsmittel unter Berücksichtigung der benötigten Rücklaufverhältnisse pro Kilogramm hergestellten Süßstoffs bis zu 100 Kilogramm Lösungsmittel verdampft werden.
Bei den bisher üblichen Methoden erfolgt die Reinigung des Lösungsmittels in mehr als zwei Destillationskolonnen. Hierbei muß das in der letzten Kolonne anfallende Sumpfmaterial, das Trialkylamin, höhersiedende Komponenten, wie Aceton, undefinierte Hochsieder, Festrückstände, wie Harze und geringe Mengen des Salzes der Süßsäure, und noch etwa 70 % Lösungsmittel als Lösungsvermittler für die Festrückstände enthält, verbrannt werden. Eine weitere Eindampfung, d.h. eine weitergehende Rückgewinnung von Lösungsmittel ist nicht möglich, weil sonst Anbackungen von Feststoffen auftreten, die zu mechanischen Störungen führen .

Im Hinblick auf die Nachteile dieses bekannten Verfahrens bestand daher die Aufgabe insbesondere darin, ein Verfahren zur Herstellung von Acesulfamsalzen bereitzustellen, das eine einfachere Rückgewinnung des eingesetzten Lösungsmittels aus dem rohen Lösungsmittel unter Einsatz von höchstens zwei Kolonnen erlaubt und die Verbrennung der anfallenden Nebenprodukte und Rückstände zumindest weitgehend vermeidet.

Es wurde nun ein Verfahren gefunden, mit dem die Ausschleusung lösungsmittelhaltiger Sumpfströme vermieden wird und das es zusätzlich erlaubt, die Lösungsmittelrückgewinnung zu reinem, für die Herstellung der Acetoacetamidoverbindung (I) und für die Herstellung der Mischung mit SO₃ geeignetem Lösungsmittel in wahlweise einer oder höchstens zwei Destillationskolonnen, unter Wegfall einer speziellen Destillationskolonne zur Abtrennung von Hochsiedern und Rückständen durchzuführen.

Die vorliegende Erfindung betrifft daher ein Verfahren zur Herstellung von Acesulfamsalzen durch Umsatz von Amidosulfonsäuresalzen mit Diketen zu einem Acetoacetamidosulfonsäuresalz (I), Ringschluß durch Einwirkung von mindestens etwa der äquimolaren Menge SO₃, wobei zumindest diese Ringschlußreaktion in Gegenwart eines halogenierten, aliphatischen Kohlenwasserstoffes als inertem Lösungsmittel erfolgt, Behandlung des Cyclisierungsproduktes (der organischen Phase) mit Wasser und Überführung des erhaltenen Acesulfams-H (II) in die Form eines nicht-toxischen Salzes (III), dadurch gekennzeichnet, daß bei der destillativen Aufarbeitung des anfallenden rohen Lösungsmittels, nach Abtrennung von Wasser und Leichtsiedern und Rückgewinnung von für den Wiedereinsatz bei der Herstellung der Verbindungen (I) und/oder (II) ausreichend reinem Lösungsmittel, der verbleibende, lösungsmittelhaltige Destillationsrückstand direkt ohne weitere Reinigung in das System nach dem Reaktionsgefäß zur Durchführung der Ringschlußreaktion zurückgeführt wird.

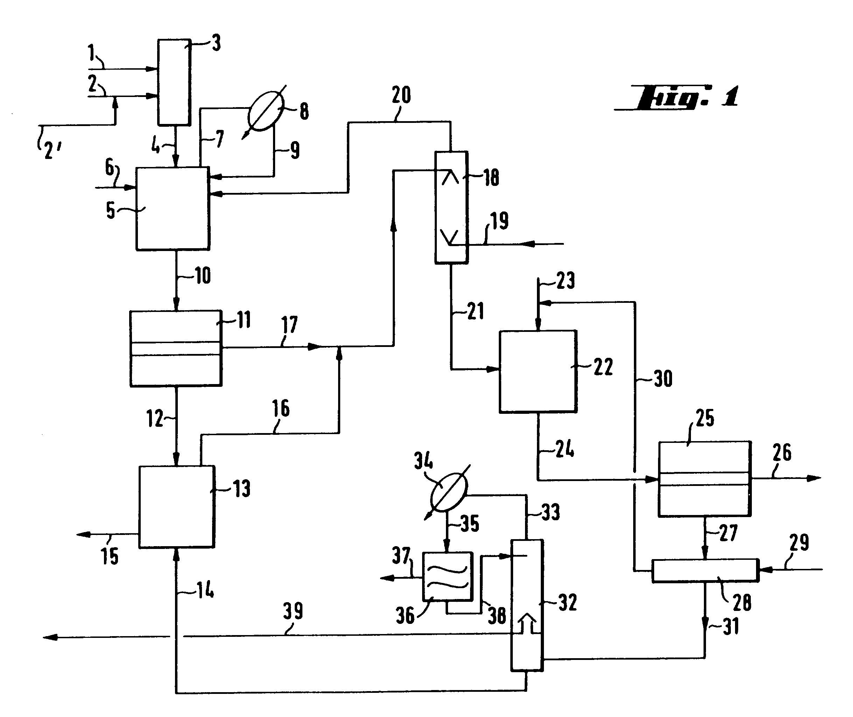

Vorzugsweise wird dieser lösungsmttelhaltige Destillationsrückstand in das Gefäß zur Behandlung der organischen Phase mit Wasser (Hydrolysegefäß (5)), in das Phasentrenngefäß (11) und/oder in das Extraktionsgefäß (13) zurückgeführt; siehe hierzu die nachfolgende Figur 1.

Die Herstellung des Acesulfams-Salzes, vorzugsweise Acesulfams-K (III), erfolgt in an sich bekannter Weise, wie in den genannten EP-Offenlegungsschriften 155.634, 159.516, 217.024 und 218.076 beschrieben, auf die hier Bezug genommen wird. Dementsprechend wird zunächst die Acetoacetamidoverbindung (I) durch Umsatz von vorzugsweise eines Salzes der Amidosulfonsäure, insbesondere des Trialkylammoniumsalzes, bei Temperaturen von zumeist 0 bis 100°C, vorzugsweise von 20 bis 45°C, mit Diketen in einem inerten Lösungsmittell vorzugsweise gleichfalls ein halogenierter, aliphatischer Kohlenwasserstoff, insbesondere Methylenchlorid hergestellt. Danach wird mit SO₃, das vorzugsweise in einer mehr als äquimolaren Menge eingesetzt wird, die Ringschlußreaktion zu einem SO₃-Addukt als einem nicht weiter isolierten Zwischenprodukt durchgeführt, wobei als Lösungsmittel vorzugsweise das gleiche wie in der ersten Stufe, insbesondere Methylenchlorid eingesetzt wird. Die Temperaturen liegen hier im allgemeinen zwischen -20 und 40°C, vorzugsweise -10 und 15°C. Dieses SO₃-Addukt wird anschließend zur Süßstoffsäure (II) hydrolysiert, wobei hier die Temperaturen in der Regel bei 20 bis 50°C, vorzugsweise 35 bis 45°C liegen. Diese Süßstoffsäure wird dann mit einer geeigneten Lauge, vorzugsweise Kalilauge, in einer Neutralisationsstufe in ein nicht-toxisches Salz (III), vorzugsweise das Kaliumsalz, übergeführt.

Bei den als Lösungsmittel eingesetzten halogenierten, aliphatischen Kohlenwasserstoffen handelt es sich vorzugsweise um solche mit bis zu 4 C-Atomen, die mit Wasser ein Azeotrop bilden, wie z. B. Methylenchlorid, Chloroform, 1,2-Dichlorethan, Trichlorethylen, Tetrachlorethylen, Trichlor-fluorethylen etc., wobei Methylenchlorid besonders bevorzugt ist.

In einer bevorzugten Ausführungsform der Erfindung wird sowohl für das Acetoamid-N-sulfonsäuresalz (I) als auch für das SO₃ das gleiche Lösungsmittel, vorzugsweise Methylenchlorid, verwendet.

Das Molverhältnis von Acetoacetamid-N-sulfonat (I) zu SO₃ kann zwar etwa 1:1 sein, bevorzugt ist aber ein bis zu etwa 20-facher SO₃-Überschuß, vorzugsweise ein etwa 3- bis 10-facher, insbesondere etwa 4- bis 7-facher molarer Überschuß.

Im bevorzugten Fall des Einsatzes von Acetoacetamid-N-sulfonsäuresalzen (I) und SO₃ im Molverhältnis 1 zu mehr als 1 entsteht bei der Ringschlußreaktion ein "SO₃-Addukt", aus dem das Acesulfam-H(II) durch Hydrolyse freigesetzt werden muß. Diese Hydrolyse erfolgt durch Zugabe von Wasser oder Eis und/oder Zugabe von verdünnter wäßriger Schwefelsäure zweckmäßig in einer - im Verhältnis zum angewandten SO₃-Überschuß - etwa 2- bis 6-fachen molaren Menge.
Vorzugsweise wird für diese Hydrolyse sowohl Wasser als auch verdünnte wäßrige Schwefelsäure eingesetzt, die aus dem nachgeschalteten Extraktor (18) stammt und die im allgemeinen eine Konzentration von 2 bis 20 Gew.-% aufweist.

Nach der Hydrolyse liegt dann ein 2- oder (wenn bereits Acesulfam-H ausgefallen ist) 3-Phasengemisch vor. Das Acesulfam-H (II) befindet sich im wesentlichen gelöst in der organischen und in der Schwefelsäurephase. Die organische Phase wird dann abgetrennt.
Die von der wäßrig-schwefelsäuren Phase abgetrennte organische Phase bzw. die entsprechenden vereinigten organischen Phasen werden vorzugsweise in dem genannten Extraktor (18) dann durch Extraktion mit Wasser gereinigt, wobei in erster Linie die hierin enthaltene Schwefelsäure aus der organischen Phase extrahiert wird.

Das Volumenverhältnis der organischen Phase zur wäßrigen Extraktionsphase liegt im allgemeinen bei etwa (20-5) 1. Auch mit wesentlich geringeren Wassermengen kann jedoch oft noch eine effektvolle Reinigung erzielt werden.

Die Extraktion erfolgt im einfachsten Fall durch Rühren der beiden Phasen in einem Rührkolben oder einem Rührkessel; als spezielle Vorrichtungen kommen im Prinzip alle technischen Extraktionsapparate in Frage wie z.B. Mixer-Settler-Apparaturen, Siebbodenkolonnen, Füllkörperkolonnen, Karr-Kolonnen etc. Auch Mischelemente wie z. B. statische Mischer können zur Intensivierung des Kontakts der Extraktionsphasen herangezogen werden.

Der Anteil an gleichzeitig mit der Schwefelsäure extrahiertem Acesulfam-H liegt im allgemeinen je nach der eingesetzten Wassermenge zwischen etwa 2 und 30 % des insgesamt in der organischen Phase enthaltenen Acesulfams-H. Für die Wirtschaftlichkeit des Gesamtverfahrens ist es von Bedeutung, die Wasserphase wieder in die Hydrolyse des "SO₃-Addukts" zurückzuführen.

Aus der gereinigten organischen Phase bzw. den gereinigten vereinigten organischen Phasen werden die nicht-toxischen Salze (III) des Acesulfams-H durch Neutralisation mit Basen gewonnen. Als Basen kommen hier diejenigen mit nicht-toxischen Kationen in Frage. Bevorzugt sind Kaliumbasen (Lösungen von KOH, KHCO₃, K₂CO₃ etc.), insbesondere KOH.

Die Neutralisation des Acesulfams-H und die Gewinnung von dessen nicht-toxischen Salzen aus der dieses Acesulfam-H enthaltenden gereinigten organischen Phase geschieht mit Vorteil durch intensiven Kontakt der gereinigten organischen Phase bzw. der entsprechenden vereinigten organischen Phasen mit wäßriger Lauge. Der intensive Kontakt erfolgt im allgemeinen nach Art und Weise einer Extraktion nach den hierfür üblichen Verfahren in den üblichen Vorrichtungen, wie sie bereits voranstehend beschrieben wurden. Auch Mischelemente, wie z. B. statische Mischer, können hier verwendet werden.

Bei der Neutralisation wird im allgemeinen so viel Base zugesetzt, bis die wäßrige Phase einen pH-Wert von etwa 5 bis 12, vorzugsweise etwa 8 bis 11 erreicht. Aus der wäßrigen Phase wird das Acesulfam-Salz dann auf übliche Weise (durch Kristallisation) gewonnen.

Gemäß einer bevorzugten Ausführungsform erfolgt die destillative Aufarbeitung des Lösungsmittels, vorzugsweise Methylenchlorid, nur in einer Destillationskolonne. Gemäß einer weiteren bevorzugten Ausführungsform wird der lösungsmittelhaltige Destillationsrückstand an mehr als eine Stelle des Systems zurückgeführt, insbesondere in das Phasentrenngefäß (11) und / oder das Extraktionsgefäß (13).

Eine weitere bevorzugte Ausführungsform sieht vor, daß man als
Acetoacetamidosulfonsäuresalz (I) Acetoacetamidosulfonsäuretrialkylammoniumsalze, gelöst in Methylenchlorid, verwendet,
daß man den Ringschluß durch die Einwirkung einer mehr als äquimolaren Menge von SO₃, gegebenenfalls in gleicher Weise in Methylenchlorid als Lösungsmittel, durchführt,
daß man das nach der Ringschlußreaktion als SO₃-Addukt anfallende undefinierte Cyclysierungsprodukt in einem Hydrolysegefäß zu Acesulfam-H hydrolysiert, daß man in einem nachgeschalteten Phasentrenngefäß eine Trennung in organische Phase und wäßrige, Schwefelsäure enthaltende Phase durchführt, diese wäßrige Phase dann in einem Extraktionsgefäß mit Methylenchlorid extrahiert und dieses Methylenchlorid, zusammen mit der organischen Phase aus dem
Phasentrenngefäß, einem weiteren Extraktor zum Auswaschen von Schwefelsäure-und Sulfatresten mit Wasser zuführt,
daß man aus der so gereinigten organischen Phase die nicht-toxischen Salze des Acesulfams-H durch Neutralisation mit Basen gewinnt und in einem nachgeschalteten Phasentrenngefäß eine Trennung in wäßrige Salzlösung und Methylenchloridphase durchführt, und
daß man diese Methylenchloridphase, ggfs. über mindestens eine weitere Extraktionsstufe, der Destillationskolonne zuführt.

Bevorzugt erfolgt die Aufarbeitung und Rückgewinnung des Lösungsmittels (Methylenchlorids) im Rahmen des erfindungsgemäßen Verfahrens in der Weise, daß
das rohe Lösungsmittel in eine Destillationskolonne in deren Sumpf oder oberhalb von deren Sumpf eingeleitet wird,
von dieser Destillationskolonne der Destillationsrückstand, der neben Teilen des Lösungsmittels feste Nebenprodukte sowie Hochsieder enthält, abgezogen und in das System, vorzugsweise in das Hydrolysegefäß (5), das Phasentrenngefäß (11) und/oder in das Extraktionsgefäß (13) zur Extraktion der Schwefelsäurephase zurückgeführt wird,
am Kopf dieser Destillationskolonne das Wasser, geringe Mengen des Lösungsmittels und mit diesem die gegebenenfalls vorhandenen Leichtsieder abgezogen werden, während der Hauptteil des Lösungsmittels als Rücklauf in die Destillationskolonne zurückgeführt wird, und
vom Mittelteil der Kolonne von einem Zwischenboden, der sich unterhalb des Rücklaufes und oberhalb des Zulaufs des in die Kolonne eingegebenen rohen Lösungsmittels befindet, ausreichend gereinigtes Lösungsmittel abzieht und dieses wieder in das System zurückführt, vorzugsweise in die Reaktionsgefäße zur Herstellung der Verbindungen (I) und/oder (II) sowie zur Herstellung der SO₃-Lösung. Selbstverständlich können auch Teile dieses gereinigten Lösungsmittels, das im allgemeinen höchstens 2 Gew.-%, vorzugsweise höchstens 1 Gew.-% und insbesondere nur noch 0,9 bis 0,1 Gew.-% an Verunreinigungen enthält, auch in nachfolgende Stellen des Systems eingebracht werden.

Das erfindungsgemäße Verfahren bietet überraschende Vorteile. So wird der apparative Aufwand erheblich geringer, da nur noch eine Kolonne oder höchstens zwei Kolonnen anstelle von drei Kolonnen benötigt werden. Weiterhin fallen keine lösungsmittelhaltigen Rückstände an, für deren Entsorgung (Verbrennung) ansonsten Sondereinrichtungen notwendig sind. Außerdem wird der Energieverbrauch für die destillative Reinigung des rohen Lösungsmittels geringer.

Die Durchführbarkeit des erfindungsgemäßen Verfahrens war überraschend. Denn bei Rückführung der Nebenprodukte, wie Aceton und polymere Feststoffe, in das System war eine Aufkonzentrierung im Gesamtkreislauf zu erwarten gewesen, die schließlich bis zum Endprodukt (Acesulfam-Salz(K)-Lösung) durchschlagen sollte. Verunreinigungen in der Acesulfam-Salz(K)-Lösung, die weiter zu reinem festen Acesulfam-Salz(K) aufgearbeitet wird, erschweren dessen Reingewinnung oder machen die Reingewinnung, wenn es sich um die in Lösung befindlichen, stark gefärbten Polymere handelt, unmöglich.

Weiterhin war zu erwarten gewesen, daß in der beschriebenen Destillationskolonne das von dem Zwischenboden abgezogene Lösungsmittel wesentlich mehr Wasser enthält als tatsächlich gefunden wurde; der Wassergehalt liegt in dem Lösungsmittel des Abzugs vom Zwischenboden aber überraschenderweise wesentlich niedriger als im Sumpf und im Kopf der Kolonne.

Die vorliegende Erfindung betrifft weiterhin eine Anordnung zur Durchführung des erfindungsgemäßen Verfahrens, bestehend im wesentlichen aus einem Reaktionsgefäß (3) zur Durchführung der Cyclisierungsreaktion aus den Verbindungen(I) und SO₃, einem nachgeschalteten Hydrolysegefäß (5) zur Gewinnung der Süßstoffsäure (II), einem damit verbundenen Phasentrenngefäß (11) zur Abtrennung der organischen Phase und der Schwefelsäurephase, vorzugsweise einem Extraktor (13) zur Extraktion der abgetrennten Schwefelsäurephase mit dem Lösungsmittel, vorzugsweise einem weiteren Extraktor (18) zur Extraktion der aus (11) und (13) zusammengeführten organischen Phasen mit Wasser, einem Neutralisationsgefäß (22) zur Überführung der Süßstoffsäure (II) in das entsprechende Salz (III), einem Phasentrenngefäß (25) zur Trennung der wäßrigen Lösung des Salzes (III) vom Lösungsmittel und einer Destillationskolonne (32).

Vorzugsweise ist an die Destillationskolonne (32) ein Phasentrenngefäß (36) angeschlossen, in welches das Brüdenkondensat vom Kolonnenkopf übergeht und hier in eine wäßrige Phase und eine Lösungsmittelphase getrennt wird. Letztere wird wieder in die Kolonne zurückgeführt. Von einem Zwischenboden der Kolonne, die im allgemeinen 15 bis 50, vorzugsweise 20 bis 30 theoretische Böden aufweist, wird reines Lösungsmittel abgezogen. Vom Kolonnenboden (Sumpf) wird der lösungsmittelhaltige Destillationsrückstand vorzugsweise in das Hydrolysegefäß (5), in das Phasentrenngeäß (11) und/oder in das Extraktionsgefäß (13) zurückgeführt. Dieser Destillationsrückstand enthält im allgemeinen noch mindestens 10 Gew.-% vorzugsweise 50 bis 98 Gew.-% und insbesondere 75 bis 95 Gew.-% an Lösungsmittel.

Das erfindungsgemäße Verfahren wir nun anhand der Figur 1 beispielhaft erläutert:

Über Leitung (1) wird eine Lösung von SO₃ in Methylenchlorid dem Reaktionsgefäß (Mischer) (3) zugeführt, über Leitung (2) erfolgt die Zufuhr von Acetoacetamidotrialkylammoniumsatzlösung in Methylenchlorid zum Reaktionsgefäß (3). Über Leitung (2') wird dieser Lösung zusätzlich Methylenchlorid zur Verdünnung zudosiert. Die Reaktionswärme wird über Verdampfung eines Teils des Methylenchlorids abgeführt, verdampfendes Methylenchlorid und Flüssigprodukt gehen über Leitung (4) gemeinsam zu dem mit Rührer versehenen Gefäß (5). Über die Leitung (6) wird dem Gefäß (5) Wasser zugeführt, die Reaktionswärme auch des Hydrolyse (Hydratations)-Schrittes wird über Methylenchloridverdampfung abgeführt. Die Methylenchloridddämpfe gehen über Leitung (7) zum Kondensator (8), das Kondensat läuft über Leitung (9) zum Gefäß (5) zurück. Das Flüssiggemisch im Behälter (5) geht über Leitung (10) zum Phasentrenngefäß (11). Die untere Schwefelsäurephase am Gefäß (11) wird über Leitung (12) zum Extraktor(Mixer Settler-Gefäß) (13) gegeben, in dem diese Schwefelsäurephase mit Methylenchlorid aus Leitung (14) zur Extraktion der Süßstoffsäure ausgerührt wird.Die Schwefelsäurephase wird aus dem Extraktor (13) über Leitung (15) abgezogen. Das mit Süßstoffsäure beladene Methylenchlorid verläßt Gefäß (13) über Leitung (16). Die Methylenchloridphase aus dem Phasentrenngefäß (11) wird über Leitung (17) abgezogen und mit dem Methylenchlorid aus Leitung (16) zum Extraktor (18) gefahren. Dem Extraktor (18) wird zur Extraktion gelösten Sulfats unten über Leitung (19) Wasser zugeführt, das nach Verlassen des Extraktors (18) über Leitung (20) zum Hyrolysegefäß (5) geführt wird. Die Methylenchloridphase des Extraktors (18) wird über Leitung (21) zum gerührten Neutralisationsgefäß (22) geführt, dem über Leitung (23) wäßrige Kalilauge, deren Menge über den pH-Wert im Rührgefäß (22) gesteuert wird, zugeleitet wird. Das Gemisch wird über Leitung (24) zum Phasentrenngefäß (25) geführt. Die obere Phase ist wäßrige Acesulfam-K-Lösung, die über Leitung (26) abgezogen wird. Die untere Methylenchloridphase wird über Leitung (27) zum Mixer-Settler-Gefäß (28) geführt, in dem sie mit wenig Wasser, das über Leitung (29) dem Mixer-Settler (28) zugegeben wird, nachgewaschen wird. Das Waschwasser wird über Leitung (30) zusammen mit der wäßrigen Kalilauge zum Neutralisationsgefäß (22) geführt. Die aus dem Mixer Settler (28) abgezogene Methylenchloridphase ist rohes Methylenchlorid, das über Leitung (31) in den Sumpf der Destillationskolonne (32) geleitet wird. Die Brüden der Kolonne (32) gehen über Leitung (33) zum Kondensator (34) und das zweiphasige Kondensat über die Leitung (35) zum Phasentrenngefäß (36). Die sich abscheidende wäßrige Phase im Phasentrenngefäß (36) wird über Leitung (37) abgezogen, die Methylenchloridphase wird über Leitung (38) als Rücklauf zurückgeführt in Kolonne (32). Am Sumpf von Kolonne (32) wird das die Nebenprodukte enthaltende Methylenchlorid über Leitung (14) zur Extraktion der Süßstoffsäure aus der Schwefelsäure im Extraktor(13) abgezogen. Von einem in der Kolonne befindlichen Zwischenboden wird über Leitung (39) reines Methylenchlorid abgeführt, das zur Mischung mit SO₃ und/oder zur Herstellung der Acetoacetamidosulfonsäuretrialkylammoniumsalzlösung wieder eingesetzt wird.

### Vergleichsbeispiel 1

Es wird nur mit reinem frischen Methylenchlorid ohne Rückaufarbeitung gearbeitet. Die Mengenabgaben sind pro Stunde.

Eine Lösung von 11,0 kg SO₃ in 46,0 kg Methylenchlorid mit einer Temperatur von +5°C wird über Leitung (1) dem Reaktionsgefäß (3) zugeführt. Über Leitung (2) werden in das Reaktionsgefäß (3) 15,0 kg Methylenchloridlösung von 0°C eingeleitet, die 6,6 kg Acetoacetamidosulfonsäure-triethylammoniumsalz und 1,0 kg polymere Komponenten unterschiedlicher Zusammensetzung und nicht umgesetztes Einsatzmaterial in kleiner Menge enthält. Über Leitung (2') wird diese Lösung mit 12,0 kg Methylenchlorid verdünnt. Die Herstellung der Lösung erfolgt in einem Edelstahlrührkessel durch Vorlegen von 10,0 kg Methylenchlorid, Zugeben von 2,5 kg Amidosulfonsäure, 2,8 kg Triethylamin, 0,1 kg Essigsäure und anschließendes vorsichtiges Zugeben von 2,2 kg Diketen, nachdem die vorherigen Komponenten sich vollständig gelöst haben. Das in dem Reaktionsgefäß entstehende Gemisch, das als Lösung in Methylenchlorid ein nicht genau definierbares Cyclisierungsprodukt, gebildete Nebenprodukte und über die Reaktionswärme verdampfendes Methylenchlorid enthält, wird über Leitung (4) dem sog. Hydrolysegefäß (5) (Rührkessel) zugeführt.

Über Leitung (6) werden dem Hydrolysekessel (5) 8,0 kg Wasser zudosiert. Über Leitung (7) gehen 20,0 kg durch die Reaktionswärme verdampftes Methylenchlorid zum Kondensator (8), während das Kondensat über Leitung (9) zum Rührkessel (5) zurückfließt. Das Flüssigkeitsgemisch in Hydrolysekessel (5), der auf konstantem Stand gehalten wird, wird über Leitung (10) zum Phasentrenner (11) geführt. Die untere Schwefelsäurephase fließt über Leitung (12) zum Mixer Settler (13). Dem Mixer-Settler (13) werden über Leitung (14) 30,0 kg reines Frischmethylenchlorid zugefahren. Über Leitung (15) werden 26,0 kg Schwefelsäurephase abgezogen. Diese Schwefelsäurephase enthält 41 % Schwefelsäure, 31 % Wasser, 21 % Triethylammoniumhydrogensulfat, 0,5 % Essigsäure, 1 % Aceton, 0,1 % Süßstoffsäure, 3 % undefinierte organische Verbindungen und 1 % gelöstes Methylenchlorid. Über Leitung (16) wird die Methylenchloridphase aus dem Mixer-Settler (13) in Leitung (17) gegeben. Aus dem Phasentrenner (11) werden über Leitung (17) 100,5 kg Methylenchloridphase abgezogen, die neben anderen Komponenten 0,2 % Wasser, 2,4 % Süßstoffsäure und 0,4 % Sulfat enthält. Die vereinigten Methylenchloridströme aus den Leitungen (16) und (17) werden dem Extraktor (18) zugeführt. Unten in den Extraktor werden über Leitung (14) 2,7 kg Wasser eingefahren, das Extraktionswasser wird über Leitung (20) zum Hydrolysekessel (5) zurückgeführt. Die Methylenchloridphase des Extraktors (18) wird über Leitung (21) dem Neutralisationsgefäß (22) (Rührkessel, Wasserkühlung im Außenmantel) zugeführt; über Leitung (23) wird über pH-Wert Steuerung auf pH-Wert 9 im Kessel (22) ca. 10 %ige Kalilauge in den Kessel (22) gefahren. Das entstehende zweiphasige Gemisch wird über Leitung (24) zum Phasentrenner (25) geführt. Aus dem Phasentrenner (25) wird die untere Methylenchloridphase über Leitung (27) dem Mixer-Settler (28) zugeführt, in dem diese Methylenchloridphase über Leitung (29) mit 6,4 1 Wasser ausgewaschen wird. Das Waschwasser geht über Leitung (30) zusammen mit der Kalilauge zum Neutralisationsgefäß (22).

Über Leitung (31) werden 99,0 kg Methylenchlorid abgezogen. Das Methylenchlorid enthält 0,15 % Wasser, 0,1 % Triethylamin, 0,4 % Aceton und 0,5 % Polymere.

Als obere Phase des Phasentrenners (25) werden über Leitung (26) 17,6 kg Acesulfam-K-Lösung abgezogen. Die hellgelbe Lösung enthält 1 % Methylenchlorid, 17 % Acesulfam-K, 0,3 % Kaliumsulfat und 0,5 % Aceton, 0,1 % Triethylamin und < 0,5 % unbekannte Verbindungen.

Beim Eindampfen dieser Süßstofflösung auf ca. 30 % des ursprünglichen Volumens, Abkühlen und Filtration wird farbioses Acesulfam-K als Feststoff erhalten.

### Vergleichsbeispiel 2

Zufuhr von rohem Methylenchlorid zur Reaktion durch Verdünnung der Acetoacetamidosulfonsäuretriethylammoniumsalzlösung mit rohem anstelle von reinem Methylenchlorid.

Die Versuchsanordnung und Mengen sind wie in Vergleichsbeispiel 1, zur Verdünnung der Acetoacetamidoverbindung in Leitung (2) wird jedoch anstelle von reinem Methylenchlorid über Leitung (2') mit 12,0 kg rohem Methylenchlorid aus Leitung (31) in Vergleichsbeispiel 1 verdünnt.

Beim Eindampfen der über Leitung (26) erhaltenen Acesulfam-K-Lösung, die jetzt dunkel gefärbt ist, entsprechend Vergleichsbeispiel 1 wird als Feststoff gelb gefärbtes Acesulfam-K erhalten, das nur über erneutes Auflösen und weitere Behandlung der Lösung mit Adsorptionsmitteln farblos erhalten werden kann.

### Beispiel 3

Die Versuchsanordnung ist wie in den Vergleichsbeispielen. Das Rohmethylenchlorid aus dem Mixer Settler (28) wird jedoch nunmehr über Leitung (31) in den Sumpf der Destillationskolonne (32) zur Wiederaufarbeitung geführt.

Über Leitung (31) werden 99,0 kg Rohmethylenchlorid in den Sumpf der Kolonne (32) (45 Siebböden, Zwischenboden zur Flüssigkeitsabnahme am Boden 30) eingeführt.

Das Rohmethylenchlorid entspricht in der Zusammensetzung dem Rohmethylenchlorid in Vergleichsbeispiel 1, auch die anderen Einzelströme entsprechen zunächst den Einzelströmen von Vergleichsbeispiel 1.

Die Sumpfheizung von Kolonne (32) wird so einreguliert, daß über Brüdenleitung (33) in Kondensator (34) (Kühlung:Kaltwasser) 110,0 kg Destillat anfallen, die über Leitung (35) zum Phasentrenner (36) geführt werden. Über Leitung (37) werden 0,13 kg Wasserphase abgezogen. Die untere organische Methylenchloridphase wird über Leitung (38) als Rücklauf in die Kolonne zurückgeführt Am Zwischenboden werden über Leitung (39) 66,0 kg reines Methylenchlorid abgezogen. Dieses Methylenchlorid enthält als Verunreinigung 140 ppm Wasser, 40 ppm Aceton, < 30 ppm Triethylamin; es wird wieder eingesetzt als Lösungsmittel zur Herstellung der Acetoacetamidoverbindung und zur Verdünnung der Lösung vor der Reaktion sowie zur Herstellung der SO₃-Lösung. .

Aus Sumpf von Kolonne (32) werden über Leitung (14) 29,7 kg Material abgezogen, das zur Extraktion der Süßstoffsäure aus der Schwefelsäurephase im Mixer-Settler Apparat (13) verwendet und über Leitung (16) wieder der Methylenchloridphase über Leitung (17) zugeführt wird.

Das Sumpfmaterial aus der Kolonne (32) in Leitung (14) besteht im wesentlichen aus Methylenchlorid, das 300 ppm Wasser, 1,4 % Aceton, 0,3 % Triethylamin und 1,7 % Polymere enthält.

Nach mehrtägiger Versuchsdauer, wenn alle Einzelströme im Konzentrationsgleichgewicht sind, zeigt der Sumpfstrom (14) einen geringen Anstieg der Acetonkonzentration auf 2 %, eine Ankonzentrierung der Polymeren oder anderer Komponenten tritt jedoch nicht auf. Die abgezogene Schwefelsäure enthält 2 % Aceton, 0,5 % Kondensationsprodukte des Acetons, 5 % Polymere und undefinierte organische Verbindungen.

Über Leitung (26) werden 17,6 kg Acesulfam-K-Lösung abgezogen, die hellgelbe Lösung enthält 17 % Acesulfam-K, 1 % Methylenchlorid, 0,3 % Kaliumsulfat, 0,6 % Aceton, 0,1 % Triethylamin, < 0,5 % unbekannte Verbindungen. Beim Eindampfen dieser Lösung auf 30 % des ursprünglichen Volumens, Abkühlen und Filtration wird farbloses Acesulfam-K als Feststoff erhalten.

Im Prinzip ein gleiches Ergebnis wird erhalten, wenn man den Sumpf der Kolomne (32) dem Hydrolysegefäß (5) zuführt. In diesem Fall kann zur Extraktion der Süßstoffsäure aus der Schwefelsäurephase im Gefäß (13) auch z.B. Kopfprodukt von Kolomne (32) verwendet werden. Der Phasentrenner (32) ist bei dieser Variante nicht unbedingt erforderlich.

## Patentansprüche

1. Verfahren zur Herstellung von Acesulfamsalzen durch Umsatz von Amidosulfonsäuresalzen mit Diketen zu einem Acetoacetamidosulfonsäuresalz (I), Ringschluß durch Einwirkung von mindestens etwa der äquivalenten Menge SO₃, wobei zumindest diese Ringschlußreaktion in Gegenwart eines halogenierten, aliphatischen Kohlenwasserstoffes als inertem Lösungsmittel erfolgt, Behandlung des Cyclisierungsproduktes mit Wasser und Überführung des erhaltenen Acesulfams-H (II) in die Form eines nicht-toxischen Salzes, dadurch gekennzeichnet, daß bei der destillativen Aufarbeitung des anfallenden rohen Lösungsmittels, nach Abtrennung von Wasser und Leichtsiedern und Rückgewinnung von für den Wiedereinsatz bei der Herstellung der Verbindungen (I) und/oder (II) ausreichend reinem Lösungsmittel, der verbleibende lösungsmittelhaltige Destillations-rückstand direkt ohne weitere Reinigung ins System nach dem Reaktionsgefäß zur Durchführung der Ringschlußreaktion zurückgeführt wird.

2. Verfahren nach Anspruch 1, dadurch gekennzeichnet, daß als Lösungsmittel Methylenchlorid eingesetzt wird.

3. Verfahren nach Anspruch 1 oder 2, dadurch gekennzeichnet, daß die destillative Aufarbeitung nur in einer Destillationskolonne erfolgt.

4. Verfahren nach mindestens einem der Ansprüche 1 bis 3, dadurch gekennzeichnet, daß der lösungsmittelhaltige Destillationsrückstand mindestens an eine Stelle des Systems, vorzugsweise in das Gefäß zur Behandlung des Cyclisierungsproduktes mit Wasser, das diesem nachgeschalteten Phasentrenngefäß und/oder diesem folgenden Extraktionsgefäß, zurückgeführt wird.

5. Verfahren nach mindestens einem der Ansprüche 1 bis 4, dadurch gekennzeichnet, daß man als Acetoacetamidosulfonsäuresalz Acetoacetamidosulfonsäuretrialkylammoniumsalze verwendet, daß man den Ringschluß durch die Einwirkung einer mehr als äquimolaren Menge an SO₃, vorzugsweise in Methylenchlorid als Lösungsmittel durchführt,
daß man das nach der Ringschlußreaktion anfallende SO₃-Addukt in einem Hydrolysegefäß zu Acesulfam-H hydrolysiert,
daß man in einem nachgeschalteten Phasentrenngefäß eine Trennung in organische Phase und wäßrige, Schwefelsäure enthaltende Phase durchführt, diese wäßrige Phase dann in einem Extraktionsgefäß mit Methylenchlorid extrahiert und dieses Methylenchlorid, zusammen mit der organischen Phase aus dem Phasentrenngefäß, einem weiteren Extraktor zum Auswaschen mit Wasser zuführt,
daß man aus der so gereinigten organischen Phase die nicht-toxischen Salze des Acesulfams-H durch Neutralisation mit Basen gewinnt und in einem nachgeschalteten Phasentrenngefäß eine Trennung in wäßrige Salzlösung und Methylenchloridphase durchführt, und
daß man diese Methylenchloridphase, gegebenenfalls über mindestens eine weitere Extraktionsstufe, der Destillationskolonne zuführt.

6. Verfahren nach mindestens einem der Ansprüche 1 bis 5, dadurch gekennzeichnet,
daß das rohe Lösungsmittel in eine Destillationskolonne in deren Sumpf oder oberhalb von diesem Sumpf eingeleitet wird,
von dieser Destillationskolonne der Destillationsrückstand, der neben Teilen des Lösungsmittels feste Nebenprodukte sowie Hochsieder enthält, abgezogen und in das Gefäß zur Behandlung des Cyclisierungsproduktes in das diesem nachgeschalteten Phasentrenn-gefäß und/oder in das diesem folgenden Extraktionsgefäß zur Extraktion der Schwefelsäurephase zurückgeführt wird,
am Kopf dieser Destillationskolonne das Wasser, geringe Mengen des Lösungsmittels und mit diesem die gegebenenfalls vorhandenen Leichtsieder abgezogen werden, während der Hauptteil des Lösungsmittels als Rücklauf in die Destillationskolonne zurückgeführt wird
und vom Mittelteil der Kolonne von einem Zwischenboden, der sich unterhalb des Rücklaufs und oberhalb des Zulaufs des in die Kolonne eingegebenen rohen Lösungsmittels befindet, gereinigtes Lösungsmittel abzieht und dieses wieder in das System zurückführt.

7. Verfahren nach Anspruch 6, dadurch gekennzeichnet, daß das gereinigte Lösungsmittel in das Reaktionsgefäß zurückgeführt wird.

8. Verfahren nach mindestens einem der Ansprüche 1 bis 7, dadurch gekennzeichnet, daß der lösungsmittelhaltige Destillationsrückstand in das Hydrolysegefäß zurückgeführt wird.

9. Verfahren nach mindestens einem der Ansprüche 1 bis 8, dadurch gekennzeichnet, daß der lösungsmittelhaltige Destillationsrückstand in den hinter dem Hydrolysegefäß vorhandenen Extraktor zur Extraktion der in der wäßrigen Phase noch vorhandenen Acesulfam-H-Verbindung zurückgeführt wird.

10. Verfahren nach mindestens einem der Ansprüche 1 bis 9, dadurch gekennzeichnet, daß der Destillationsrückstand aus der Destillationskolonne mindestens noch 10 Gew.-%, vorzugsweise bis zu 98 Gew.-%, an Lösungsmittel enthält.

11. Anordnung zur Durchführung des erfindungsgemäßen Verfahrens, bestehend im wesentlichen aus einem Reaktionsgefäß (3) zur Durchführung der Cyclisierungsreaktion aus einem Acetoacetamidosulfonsäuresalz(I) und SO₃, einem nachgeschalteten Hydrolysegefäß (5) zur Gewinnung der Süßsäure (II), einem damit verbundenen Phasentrenngefäß (11) zur Abtrennung der organischen Phase und der Schwefelsäurephase, einem Neutralisationsgefäß (22) zur Überführung der Süßstoffsäure (II) in das entsprechende Salz (III), einem Phasentrenngefäß (25) zur Trennung der wäßrigen Lösung des Salzes (III) vom Lösungsmittel und einer Destillationskolonne (32).

12. Anordnung nach Anspruch 11, dadurch gekennzeichnet, daß sie zusätzlich einen Extraktor (13) zur Extraktion der abgetrennten Schwefelsäurephase mit dem Lösungsmittel sowie einen weiteren Extraktor (18) zur Extraktion der aus (11) und (13) zusammengeführten organischen Phasen mit Wasser aufweist.

13. Anordnung nach Anspruch 11 oder 12, dadurch gekennzeichnet, daß an die Destillationskolonne (32) ein Phasentrenngefäß (36) angeschlossen ist, in das die kondensierten Brüden vom Kolonnenkopf übergehen und in dem eine Trennung in eine wäßrige Phase und in eine Lösungsmittelphase erfolgt.

14. Anordnung nach mindestens einem der Ansprüche 11 bis 13, dadurch gekennzeichnet, daß die Destillationskolonne (32) 15 bis 50 theoretische Böden aufweist.

## Claims

1. A process for the preparation of acesulfam salts by reaction of salts of amidosulfonic acid with diketene to give a salt of acetoacetamidosulfonic acid (I), ring closure by the action of at least about an equivalent amount of SO₃, at least this ring closure reaction being carried out in the presence of a halogenated, aliphatic hydrocarbon as an inert solvent, treatment of the cyclization product with water and conversion of the resulting acesulfam-H (II) into the form of a non-toxic salt, which comprises, in the work-up by distillation of the resulting crude solvent, after removal of water and low-boilers and recovery of solvent sufficiently pure for reuse in the preparation of compounds (I) and/or (II), directly returning the remaining, solvent-containing distillation residue, without further purification, into the system downstream of the reaction vessel for carrying out the ring closure reaction.

2. The process as claimed in claim 1, wherein the solvent used is methylene chloride.

3. The process as claimed in claim 1 or 2, wherein the work-up by distillation is carried out in only one distillation column.

4. The process as claimed in at least one of claims 1 to 3, wherein the solvent-containing distillation residue is returned at least at one point of the system, preferably into the vessel for treating the cyclization product with water, the phase separation vessel downstream of this and/or the extraction vessel following this.

5. The process as claimed in at least one of claims 1 to 4, wherein the salt of acetoacetamidosulfonic acid used is trialkylammonium acetoacetamido-sulfonate,
the ring closure is carried out by the action of a more than equimolar amount of SO₃, preferably in methylene chloride as solvent,
the SO₃ adduct produced after the ring closure reaction is hydrolyzed in a hydrolysis vessel to give acesulfam-H,
a separation is carried out in a downstream phase separation vessel into an organic phase and aqueous sulfuric acid-containing phase, this aqueous phase is then extracted in an extraction vessel with methylene chloride and this methylene chloride, together with the organic phase from the phase separation vessel, is fed to a further extractor for washing with water,
the non-toxic salts of acesulfam-H are recovered, by neutralization using bases, from the organic phase thus purified and a separation is carried out in a downstream phase separation vessel into aqueous salt solution and a methylene chloride phase, and
this methylene chloride phase is fed, if desired via at least one further extraction stage, to the distillation column.

6. The process as claimed in at least one of claims 1 to 5, wherein
the crude solvent is introduced into a distillation column in the bottom thereof or above this bottom, the distillation residue, which in addition to parts of the solvent contains solid by-products and high-boilers, is withdrawn from this distillation column and returned into the vessel for the treatment of the cyclization product, into the phase separation vessel downstream of this and/or into the extraction vessel following this for extraction of the sulfuric acid phase,
the water, small amounts of the solvent and any low-boilers which may be present with this are withdrawn at the head of this distillation column, while the majority of the solvent is returned as reflux into the distillation column,
and purified solvent is withdrawn from the central part of the column from an intermediate plate which is located beneath the reflux and above the feed of the crude solvent introduced into the column and this purified solvent is returned into the system.

7. The process as claimed in claim 6, wherein the purified solvent is returned into the reaction vessel.

8. The process as claimed in at least one of claims 1 to 7, wherein the solvent-containing distillation residue is returned into the hydrolysis vessel.

9. The process as claimed in at least one of claims 1 to 8, wherein the solvent-containing distillation residue is returned into the extractor, provided downstream of the hydrolysis vessel, for the extraction of the acesulfam-H compound still present in the aqueous phase.

10. The process as claimed in at least one of claims 1 to 9, wherein the distillation residue from the distillation column still contains at least 10% by weight, preferably up to 98% by weight, of solvent.

11. An arrangement for carrying out the process according to the invention, essentially comprising a reaction vessel (3) for carrying out the cyclization reaction from a salt of acetoacetamidosulfonic acid (I) and SO₃, a downstream hydrolysis vessel (5) for the recovery of the sweetener acid (II), a phase separation vessel (11) connected thereto for the separation of the organic phase and the sulfuric acid phase, a neutralization vessel (22) for the conversion of the sweetener acid (II) into the corresponding salt (III), a phase separation vessel (25) for the separation of the aqueous solution of the salt (III) from the solvent and a distillation column (32).

12. The arrangement as claimed in claim 11, which additionally has an extractor (13) for the extraction of the separated sulfuric acid phase using the solvent and a further extractor (18) for the extraction of the organic phases combined from (11) and (13) using water.

13. The arrangement as claimed in claim 11 or 12, wherein a phase separation vessel (36) is connected to the distillation column (32), into which phase separation vessel the condensed vapors pass from the column head and in which a separation is carried out into an aqueous phase and into a solvent phase.

14. The arrangement as claimed in at least one of claims 11 to 13, wherein the distillation column (32) has 15 to 50 theoretical plates.

## Revendications

1. Procédé pour la préparation de sels d' acésulfame par mise en réaction de sels d'acide amidosulfonique avec du dicétène, pour l'obtention d'un sel d'acide acétoacétamidosulfonique (I), cyclisation sous l'effet d'au moins environ la quantité équivalente de SO₃, au moins cette réaction de cyclisation étant effectuée en présence d'un hydrocarbure aliphatique halogéné en tant que solvant inerte, traitement du produit de cyclisation par de l'eau et conversion de 1' acesulfame-H (II) obtenu en la forme d'un sel non toxique, caractérisé en ce que, lors du traitement final par distillation du solvant brut produit, après séparation de l'eau et des substances à bas point d'ébullition, et récupération de solvant suffisamment pur pour la réutilisation dans la préparation des composés (I) et/ou (II), le résidu de distillation contenant du solvant résiduel est directement recyclé, sans être purifié davantage, dans le système faisant suite au récipient de réaction, pour la mise en oeuvre de la réaction de cyclisation.

2. Procédé selon la revendication 1, caractérisé en ce que l'on utilise comme solvant le chlorure de méthylène.

3. Procédé selon la revendication 1 ou 2, caractérisé en ce que le traitement final par distillation s'effectue seulement dans une colonne de distillation.

4. Procédé selon au moins l'une des revendications 1 à 3, caractérisé en ce que le résidu de distillation contenant du solvant est recyclé au moins en un point du système, de préférence dans le récipient destiné au traitement du produit de cyclisation par de l'eau, le récipient de séparation de phases raccordé à la suite de celui-ci et/ou le récipient d'extraction faisant suite à celui-ci.

5. Procédé selon au moins l'une des revendications 1 à 4, caractérisé en ce que l'on utilise comme sel d'acide acétoacétamidosulfonique des acétoacétamido-sulfonates de trialkylammonium,
en ce que l'on effectue la cyclisation sous l'effet d'une quantité plus qu'équimolaire de SO₃, de préférence dans du chlorure de méthylène en tant que solvant,
en ce que l'on hydrolyse dans un récipient d'hydrolyse l'adduct de SO₃, produit après la réaction de cyclisation, en acésulfam-H,
en ce que, dans un récipient de séparation de phases raccordé à la suite, on effectue une séparation en phase organique et phase aqueuse contenant de l'acide sulfurique, on extrait ensuite cette phase aqueuse, dans un récipient d'extraction, avec du chlorure de méthylène, et ce chlorure de méthylène est envoyé à un autre extracteur, conjointement avec la phase organique provenant du récipient de séparation de phases, pour l'extraction par lavage avec de l'eau,
en ce que, à partir de la phase organique ainsi purifiée, on obtient les sels non toxiques de l'acésulfam-H par neutralisation avec des bases et, dans un récipient de séparation de phases raccordé à la suite, on effectue une séparation en solution aqueuse de sels et phase de chlorure de méthylène, et
en ce que cette phase de chlorure de méthylène est envoyée, éventuellement après au moins une autre étape d'extraction, à la colonne de distillation.

6. Procédé selon au moins l'une des revendications 1 à 5, caractérisé en ce que le solvant brut est envoyé dans une colonne de distillation dans la partie inférieure de celle-ci ou au-dessus de cette partie inférieure, le résidu de distillation de cette colonne de distillation, qui, outre des fractions du solvants, contient des sous-produits solides ainsi que des substances à haut point d'ébullition, est soutiré et est recyclé dans le récipient destiné au traitement du produit de cyclisation dans le récipient de séparation de phases raccordé à la suite de celui-ci et/ou dans le récipient d'extraction destiné à l'extraction de la phase d'acide sulfurique lui faisant suite,
à la tête de cette colonne de distillation, l'eau, de faibles quantités du solvant et, avec celui-ci, les substances à bas point d'ébullition éventuellement présentes, sont évacuées, tandis que la partie principale du solvant est recyclée en tant que courant de recyclage dans la colonne de distillation,
et, de la partie médiane de la colonne, à partir d'un plateau intermédiaire qui se trouve au-dessous du conduit de recyclage et au-dessus du conduit d'arrivée du solvant pur introduit dans la colonne, est déchargé du solvant purifié, et celui-ci est à nouveau recyclé dans le système.

7. Procédé selon la revendication 8, caractérisé en ce que le solvant purifié est recyclé dans le récipient de réaction.

8. Procédé selon au moins l'une des revendications 1 à 7, caractérisé en ce que le résidu de distillation contenant du solvant est recyclé dans le récipient d'hydrolyse.

9. Procédé selon au moins l'une des revendications 1 à 8, caractérisé en ce que le résidu de distillation contenant du solvant est recyclé dans l'extracteur, présent en amont du récipient d'hydrolyse, destiné à l'extraction du composé acésulfame-H encore présent dans la phase aqueuse.

10. Procédé selon au moins l'une des revendications 1 à 9, caractérisé en ce que le résidu de distillation, provenant de la colonne de distillation, contient au moins encore 10 % en poids, de préférence jusqu'à 98 % en poids, de solvant.

11. Installation pour la mise en oeuvre du procédé selon l'invention, consistant essentiellement en un récipient de réaction (3) destiné à l'exécution de la réaction de cyclisation à partir d'un sel d'acide acétoacétamido-sulfonique (I) et de SO₃, en un récipient d'hydrolyse (5) raccordé à la suite, destiné à la récupération de l'acide édulcorant (II), en un récipient de séparation de phases (11) relié à celui-ci, destiné à la séparation de la phase organique et de la phase d'acide sulfurique, en un récipient de neutralisation (22) destiné à la conversion de l'acide édulcorant (II) en le sel correspondant (III), en un récipient de séparation de phases (25) destiné à la séparation de la solution aqueuse du sel (III) d'avec le solvant, et en une colonne de distillation (32).

12. Installation selon la revendication 11, caractérisée en ce qu'elle comprend en outre un extracteur (13) destiné à l'extraction par le solvant de la phase d'acide sulfurique séparée, ainsi qu'un autre extracteur (18) destiné à l'extraction par de l'eau des phases organiques envoyées ensemble à partir de (11) et (13).

13. Installation selon la revendication 11 ou 12, caractérisée en ce qu'à la colonne de distillation (32) fait suite un récipient de séparation de phases (36), dans lequel passent les vapeurs chaudes condensées de la tête de la colonne, et dans lequel s'effectue une séparation en une phase aqueuse et une phase de solvant.

14. Installation selon au moins l'une des revendications 11 à 13, caractérisée en ce que la colonne de distillation (32) comporte de 15 à 50 plateaux théoriques.
